# EUROPÄISCHE PATENTSCHRIFT

(11) **EP 1 893 586 B1**
(45) Veröffentlichungstag und Bekanntmachung des Hinweises auf die Patenterteilung: **17.09.2008**
(21) Anmeldenummer: 06755248.9
(22) Anmeldetag: 18.05.2006
(51) Int. Cl.: C07D 239/54

(54) **VERFAHREN ZUR HERSTELLUNG VON 1-ALKYL-3-PHENYLURACILEN**
METHOD FOR PRODUCTION OF 1-ALKYL-3-PHENYLURACILS
PROCEDE POUR PRODUIRE DES 1-ALKYL-3-PHENYLURACILES

(30) Priorität: 24.05.2005 DE 102005024448
(43) Veröffentlichungstag der Anmeldung: 05.03.2008
(73) Patentinhaber: BASF SE, 67056 Ludwigshafen (DE)
(72) Erfinder: GEBHARDT, Joachim, 67157 Wachenheim (DE); LÖHR, Sandra, 67059 Ludwigshafen (DE); KEIL, Michael, 67251 Freinsheim (DE); SCHMIDT, Thomas, 67433 Neustadt (DE); WEVERS, Jan Hendrik, 67591 Hohensülzen (DE); ZECH, Helmut, 67098 Bad Dürkheim (DE); HÄBERLE, Rudolf, 68309 Mannheim (DE)
(86) Internationale Anmeldenummer: PCT/EP2006/062414
(87) Internationale Veröffentlichungsnummer: WO 2006/125746

(56) Entgegenhaltungen:
- WO-A-01/83459
- WO-A-20/05054208
- US-A- 4 943 309

## Beschreibung

Die vorliegende Erfindung betrifft ein Verfahren zur Herstellung von 1-Alkyl-3-phenyluracilen der Formel I 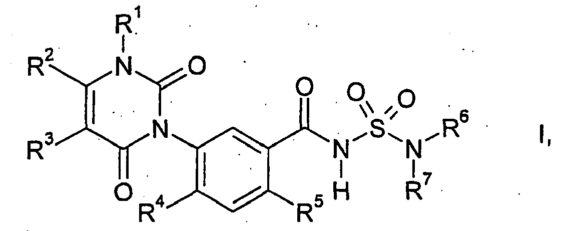 worin die Variablen die folgenden Bedeutungen haben:
- R¹: C₁-C₆-Alkyl;
- R² und R³: unabhängig voneinander
Wasserstoff, C₁-C₆-Alkyl oder C₁-C₆-Halogenalkyl;
- R⁴ und R⁵: unabhängig voneinander
Wasserstoff, Halogen, Cyano, C₁-C₆-Alkyl oder C₁-C₆-Halogenalkyl;
- R⁶ und R⁷: unabhängig voneinander
Wasserstoff, C₁-C₆-Alkyl, C₁-C₆-Alkoxy, C₃-C₆-Alkenyl, C₃-C₆-Alkynyl C₃-C₆-Cycloalkyl, C₃-C₆-Cycloalkenyl, Phenyl oder Benzyl;
indem 3-Phenyluracile der Formel II 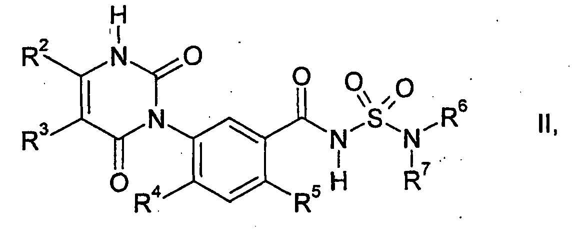 wobei die Variablen R² bis R⁷ die zuvor genannten Bedeutungen haben,
und Alkylierungsmittel der Formel III

R¹-L¹ III,

wobei R¹ die oben genannte Bedeutung hat, und
L¹ für Halogen, Hydrogensulfat, C₁-C₆-Alkylsulfat, C₁-C₆-Alkylcarbonat, C₁-C₆-Alkylsulfonyloxy, C₁-C₆-Halogenalkylsulfonyloxy oder Phenylsulfonyloxy, wobei der Phenylring einen oder mehrere Substituenten aus der Gruppe Halogen, Nitro, C₁-C₆-Alkyl und C₁-C₆-Halogenalkyl tragen kann,
steht;
miteinander umgesetzt werden,
dadurch gekennzeichnet, dass während der gesamten Umsetzung der pH-Wert durch portionsweise Basenzugabe in einem Bereich von 1 bis 6 gehalten wird.

1-Alkyl-3-phenyluracile der allgemeinen Formel I sind prinzipiell aus der WO 01/83459 bekannt. Ihre Herstellung kann gemäß der in WO 01/83459 angegebenen Lehre erfolgen.

N-Alkylierungen am freien Uracilstickstoffatom durch Umsetzung einer Uracilverbindung mit einem Alkylierungsmittel werden z.B. in der US 4,943,309 beschrieben.

Weiterhin wird die Herstellung von 1-Afkyl-3-phenyluracilen mit einer Sulfamidseitenkette in der PCT/EP/04/013615 beschrieben.

Nachteilig an diesen Vorgehensweisen ist allerdings, dass aufgrund der leichten Alkylierbarkeit der Sulfamindseitenkette Nebenreaktionen wie z.B. Alkylierung am Sulfonamidsticktoffatom bzw. Bildung von dialkylierten Produkte auftreten.
Entsprechend ist auch schon bekannt, dass Schwefelsäurediamide in einfacher Weise mit Schwefelsäurediestern oder Arensulfonsäureestern in Gegenwart einer Base alkyliert werden (z.B. R. Sowada, J. Prakt. Chem. 25, 88, 1964).

Weiterhin ist im Falle von trisubstituierten Schwefelsäurediamiden die Bildung von tetrasubstituierten Schwefelsäurediamiden bekannt (z.B. B. Unterhalt, E. Seebach, Arch. Pharm. 314, 51, 1981).

Ebenso können auch Schwefelsäurediamide, bei denen die Amidfunktion bereits einen Acylrest trägt, alkyliert werden (z.B. K. C. C. Bancroft et al., J. Heterocycl. Chem. 15, 1521, 1978; A. Martinez et al., Bioorg. Med. Chem. Lett. 9, 21, 3133, 1999).

Somit liegt der vorliegenden Erfindung die Aufgabe zugrunde, ein einfaches und wirtschaftliches Verfahren zur Herstellung von 1-Alkyl-3-phenyluracilen der Formel I bereitzustellen, durch das unerwünschte Nebenreaktionen wie z.B. die Bildung von dialkylierten Nebenprodukten unterdrückt werden, und durch das gleichzeitig hohe Ausbeuten und hohe Reinheit an Wertprodukt erzielt werden können.

Es wurde überraschenderweise gefunden, dass diese Aufgabe durch ein Verfahren gelöst wird, bei dem 3-Phenyluracile der Formel II 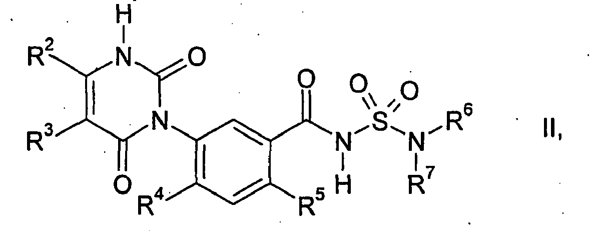 wobei die Variablen R² bis R⁶ die zuvor genannten Bedeutungen haben,
und Alkylierungsmittel der Formel III

R¹-L¹ III,

wobei R¹ die oben genannte Bedeutung hat, und
L¹ für eine nucleophil verdrängbare Abgangsgruppe steht;
miteinander umgesetzt werden,
dadurch gekennzeichnet, dass während der gesamten Umsetzung der pH-Wert durch portionsweise Basenzugabe in einem Bereich von 1 bis 6 gehalten wird.

Demnach betrifft die vorliegende Erfindung ein Verfahren zur Herstellung 1-Alkyl-3-phenyluracilen der Formel I, das die Umsetzung von 3-Phenyluracilen der Formel II und Alkylierungsmitteln der Formel III, dadurch gekennzeichnet, dass während der gesamten Umsetzung der pH-Wert durch portionsweise Basenzugabe in einem Bereich von 1 bis 6 gehalten wird, umfaßt.

Nach dem erfindungsgemäßen Verfahren werden 1-Alkyl-3-phenyluracile der Formel I in hohen Ausbeuten und hohen Reinheiten erhalten.
Dies überrascht angesichts der Tatsache, dass das eingesetzte 3-Phenyluracil der Formel II sowohl am Uracilring als auch in der Seitenkette eine reaktive NH-Gruppe aufweist, die alkyliert werden kann.
Somit würde der Fachmann eine Vielzahl von Nebenreaktionen, z.B. die Bildung entsprechender N-Alkylsulfonamide oder Gemische aus N-Alkylsulfonamiden oder N-Alkyl substituierten Uracilen einschließlich Oligomer- oder Polymerbildung erwarten.

Die 1-Alkyl-3-phenyluracile der Formel I können je nach Substitutionsmuster ein oder mehrere Chiralitätszentren enthalten und liegen dann als Enantiomeren oder Diastereomerengemische vor. Gegenstand der Erfindung ist somit ein Verfahren zur Herstellung sowohl der reinen Enantiomere oder Diastereomere als auch deren Gemische.

Die 1-Alkyl-3-phenyluracile der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹ - R⁷ oder als Reste an Phenylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Alkoxy-, Alkenyl- und Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder lod.

Ferner bedeuten beispielsweise:
- C₁-C₄-Alkyl: z.B. Methyl, Ethyl, n-Propyl, 1-Methylethyl, n-Butyl, 1-Methylpropyl, 2-Methylpropyl und 1,1-Dimethylethyl;
- C₁-C₆-Alkyl: C₁-C₄-Alkyl wie voranstehend genannt, sowie z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl: einen C₁-C₄-Alkylrest wie vorstehend genannt, der partiell oder vollständig durch Fluor, Chlor, Brom und/oder lod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl wie voranstehend genannt, sowie z.B. 5-Fluorpentyl, 5-Chlorpentyl, 5-Brompentyl, 5-lodpentyl, Undecafluorpentyl, 6-Fluorhexyl, 6-Chlorhexyl, 6-Bromhexyl, 6-lodhexyl und Tridecafluorhexyl;
- C₃-C₇-Cycloalkyl: ein monocyclischer gesättigter Kohlenwasserstoff mit 3 bis 7 Ringgliedern, wie z.B. Cyclopropyl, Cyclobutyl, Cyclopentyl, Cyclohexyl oder Cycloheptyl;
- C₃-C₇-Cycloalkenyl: ein monocyclischer partiell ungesättigter Kohlenwasserstoff mit 3 bis 7 Ringgliedern, wie z.B. Cyctoprop-1-enyl, Cycloprop-2-enyl, Cyclobut-1-enyl, Cyclobut-2-enyl, Cyclobut-1,3-dienyl, Cyclopent-1-enyl, Cyclopent-2-enyl, Cyclopent-3-enyl, Cyclopent-2,4-dienyl, Cyclohex-1-enyl, Cyclohex-2-enyl, Cyclohex-3-enyl; Cyclohex-1,3-dienyl, Cyclohex-1,5-dienyl, Cyclohex-2,4-dienyl, oder Cyclohex-2,5-dienyl.
- C₃-C₆-Alkenyl: z.B. 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂-C₆-Alkinyl: z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
- C₁-C₄-Alkoxy: z.B. Methoxy, Ethoxy, Propoxy, 1-Methyl-ethoxy, Butoxy, 1-Methylpropoxy, 2-Methylpropoxy und 1,1-Dimethylethoxy;
- C₁-C₆-Alkoxy: C₁-C₄-Alkoxy wie voranstehend genannt, sowie z.B. Pentoxy, 1-Methylbutoxy, 2-Methylbutoxy, 3-Methoxylbutoxy, 1,1-Dimethyl-propoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Di-methylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Tri-methylpropoxy, 1,2,2-Tri-methylpropoxy, 1-Ethyl-1-methylpropoxy und 1-Ethyl-2-methylpropoxy;

In einer besonders bevorzugten Ausführungsform des erfindungsgemäßen Verfahrens weisen die Variablen R¹ bis R⁶ die folgenden Bedeutungen, und zwar jeweils für sich allein oder in Kombination, auf:
- R¹: C₁-C₄-Alkyl,
bevorzugt Methyl, Ethyl, n-Propyl, iso-Propyl,
sehr bevorzugt Methyl;
- R²: Wasserstoff, C₁-C₄-Alkyl oder C₁-C₄-Halogenalkyl,
bevorzugt Wasserstoff, Methyl oder C₁-C₄-Halogenalkyl,
sehr bevorzugt C₁-C₄-Halogenalkyl;
besonders bevorzugt Difluormethyl oder Trifluormethyl,
außerordentlich bevorzugt Trifluormethyl;
- R³: Wasserstoff, C₁-C₄-Alkyl oder C₁-C₄-Halogenalkyl,
bevorzugt Wasserstoff, Methyl oder Trifluormethyl,
sehr bevorzugt Wasserstoff;
- R⁴: Wasserstoff oder Halogen,
bevorzugt Wasserstoff, Fluor oder Chlor,
sehr bevorzugt Wasserstoff oder Fluor,
besonders bevorzugt Fluor;
- R⁵: Halogen, Cyano oder C₁-C₄-Halogenalkyl,
bevorzugt Fluor, Chlor, Cyano oder Trifluormethyl,
ebenso bevorzugt Halogen oder Cyano,
sehr bevorzugt Fluor, Chlor oder Cyano,
besonders bevorzugt Chlor oder Cyano,
außerordentlich bevorzugt Chlor;
- R⁶: Wasserstoff oder C₁-C₄-Alkyl,
ebenso bevorzugt C₁-C₆-Alkyl,
sehr bevorzugt C₁-C₄-Alkyl,
besonders bevorzugt Methyl, Ethyl, n-Propyl oder iso-Propyl, außerordentlich bevorzugt iso-Propyl;
- R⁷: Wasserstoff oder C₁-C₄-Alkyl,
ebenso bevorzugt C₁-C₆-Alkyl,
sehr bevorzugt C₁-C₄-Alkyl,
besonders bevorzugt Methyl, Ethyl, n-Propyl oder iso-Propyl, außerordentlich bevorzugt Methyl.

In einer ebenso bevorzugten Ausführungsform des erfindungsgemäßen Verfahrens weist R² folgende Bedeutungen auf:
- R²: Wasserstoff oder C₁-C₆-Halogenalkyl, bevorzugt C₁-C₆-Halogenalkyl,
sehr bevorzugt C₁-C₄-Halogenalkyl,
besonders bevorzugt Difluormethyl oder Trifluormethyl,
außerordentlich bevorzugt Trifluormethyl.

In einer ebenso bevorzugten Ausführungsform des erfindungsgemäßen Verfahrens weist R² folgende Bedeutungen auf:
- R²: C₁-C₆-Alkyl oder C₁-C₆-Halogenalkyl,
bevorzugt C₁-C₄-Alkyl oder C₁-C₆-Halogenalkyl,
sehr bevorzugt C₁-C₄-Halogenalkyl,
besonders bevorzugt Difluormethyl oder Trifluormethyl,
außerordentlich bevorzugt Trifluormethyl.

In einer ebenso bevorzugten Ausführungsform des erfindungsgemäßen Verfahrens weist R⁴ folgende Bedeutungen auf:
- R⁴: Wasserstoff oder Halogen,
bevorzugt Wasserstoff,
ebenso bevorzugt Halogen,
sehr bevorzugt Fluor oder Chlor.

In einer besonders bevorzugten Ausführungsform des erfindungsgemäßen Verfahrens lassen sich 1-Alkyl-3-phenyluracile der Formel I.a (entspricht Formel I mit R² = CF₃, R³ = H und R⁷ = CH₃), insbesondere die 1-Alkyl-3-phenyluracile I.a.1 bis I.a.24 der Tabelle 1 herstellen, wobei die Definitionen der Variablen R¹, R⁴, R⁵ und R⁶ nicht nur in Kombination miteinander sondern auch jeweils für sich betrachtet für das erfindungsgemäße Verfahren eine besondere Rolle spielen.

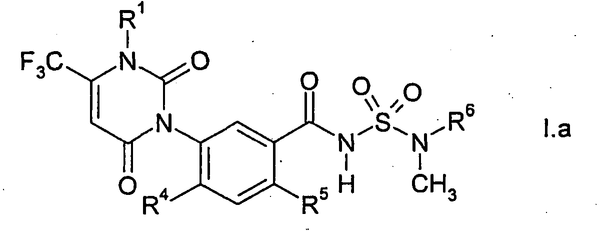

**Tabelle 1**

| Nr. | R¹ | R⁴ | R⁵ | R⁶ |
|---|---|---|---|---|
| I.a.1 | CH₃ | H | H | CH₃ |
| I.a.2 | C₂H₅ | H | H | CH₃. |
| I.a.3 | CH₃ | F | H | CH₃ |
| I.a.4 | C₂H₅ | F | H | CH₃ |
| I.a.5 | CH₃ | H | Cl | CH₃ |
| I.a.6 | C₂H₅ | H | Cl | CH₃ |
| I.a.7 | CH₃ | F | Cl | CH₃ |
| I.a.8 | C₂H₅ | F | Cl | CH₃ |
| I.a.9 | CH₃ | H | H | C₂H₅ |
| I.a.10 | C₂H₅ | H | H | C₂H₅ |
| I.a.11 | CH₃ | F | H | C₂H₅ |
| I.a.12 | C₂H₅ | F | H | C₂H₅ |
| I.a.13 | CH₃ | H | Cl | C₂H₅ |
| I.a.14 | C₂H₅ | H | Cl | C₂H₅ |
| I.a.15 | CH₃ | F | Cl | C₂H₅ |
| I.a.16 | C₂H₅ | F | Cl | C₂H₅ |
| I.a.17 | CH₃ | H | H | CH(CH₃)₂ |
| I.a.18 | C₂H₅ | H | H | CH(CH₃)₂ |
| I.a.19 | CH₃ | F | H | CH(CH₃)₂ |
| I.a.20 | C₂H₅ | F | H | CH(CH₃)₂ |
| I.a.21 | CH₃ | H | Cl | CH(CH₃)₂ |
| I.a.22 | C₂H₅ | H | Cl | CH(CH₃)₂ |
| I.a.23 | CH₃ | F | Cl | CH(CH₃)₂ |
| I.a.24 | C₂H₅ | F | Cl | CH(CH₃)₂ |

Das erfindungsgemäße Verfahren umfasst die Umsetzung von 3-Phenyluracilen der Formel II und Alkylierungsmittel der Formel III dadurch gekennzeichnet, dass während der gesamten Umsetzung der pH-Wert durch portionsweise Basenzugabe in einem Bereich von 1 bis 6 gehalten wird:

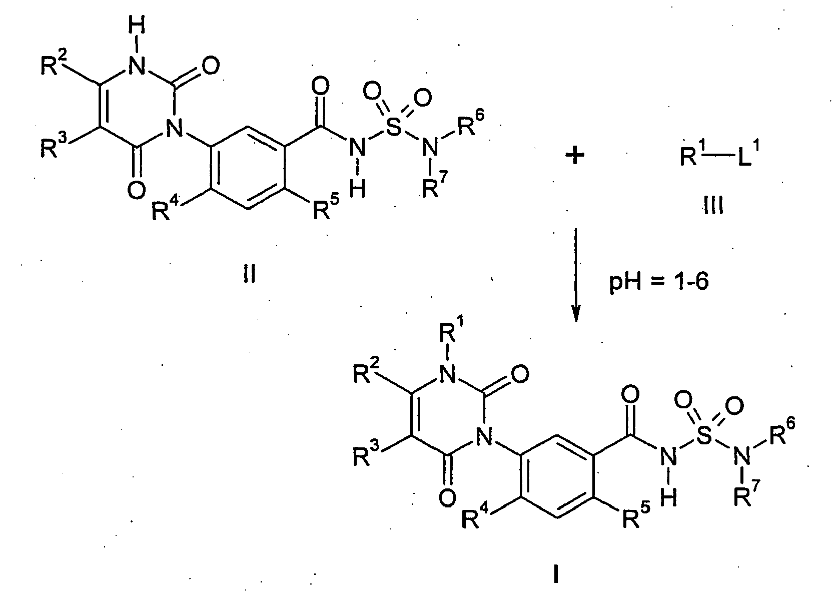

Die Gruppe L¹ im Alkylierungsmittel der Formel III steht für eine nucleophil verdrängbare Abgangsgruppe,
bevorzugt für Halogen, Hydrogensulfat, C₁-C₆-Alkylsulfat, C₁-C₆-Alkylcarbonat, C₁-C₆-Alkylsulfonyloxy, C₁-C₆-Halogenalkylsulfonyloxy oder Phenylsulfonyloxy,
wobei der Phenylring einen oder mehrere Substituenten aus der Gruppe Halogen, Nitro, C₁-C₆-Alkyl und C₁-C₆-Halogenalkyl tragen kann;
sehr bevorzugt für Halogen, C₁-C₆-Alkylsulfat, C₁-C₆-Alkylsulfonyloxy, C₁-C₆-Halogenalkylsulfonyloxy, Phenylsulfonyloxy, p-Toluolsulfonyloxy, p-Chlorphenylsulfonyloxy, p-Bromphenylsulfonyloxy oder p-Nitrophenylsulfonyloxy;
besonders bevorzugt für Halogen, C₁-C₆-Alkylsulfat, C₁-C₆-Alkylsulfonyloxy, C₁-C₆-Halogenalkylsulfonyloxy oder Phenylsulfonyloxy;
sehr bevorzugt für C₁-C₆-Alkylsulfat;
außerordentlich bevorzugt für C₁-C₄-Alkylsulfat;
ebenso besonders bevorzugt für Chlor, Brom oder lod, Methylsulfat, Methylsulfonyloxy, Trifluormethylsulfonyloxy oder Phenylsulfonyloxy.
3-Phenyluracile der Formel II sind aus WO 01/83459 und WO 04/39768 bekannt und können gemäß der zitierten Literatur hergestellt werden.

Bevorzugte Alkylierungsmittel sind C₁-C₆-Alkylhalogenide, Di-C₁-C₆-alkylsulfate, Di-C₁-C₆-alkylcarbonate, C₁-C₆-Alkylsulfonsäuren, C₁-C₆-Alkylsulfonsäure-C₁-C₄-alkylester, C₁-C₆-Halogenalkylsulfonsäuren, C₁-C₆-Halogenalkylsulfonsäure-C₁-C₄-alkylester oder Phenylsulfonsäure-C₁-C₄-alkylester,
wobei der Phenylring einen oder mehrere Substituenten aus der Gruppe Halogen, Nitro, C₁-C₆-Alkyl und C₁-C₆-Halogenalkyl tragen kann.

Sehr bevorzugte Alkylierungsmittel sind C₁-C₆-Alkylhalogenide, Di-C₁-C₆-alkylsulfate, C₁-C₆-Alkylsulfonsäure-C₁-C₄-alkylester oder Phenylsulfonsäure-C₁-C₄-alkylester.

Ebenso sehr bevorzugte Alkylierungsmittel sind C₁-C₆-Alkylhalogenide, Di-C₁-C₆-alkylsulfate, Di-C₁-C₆-alkylcarbonate, C₁-C₆-Alkylsulfonsäure-C₁-C₄-alkylester oder Phenylsulfonsäure-C₁-C₄-alkylester.

Besonders bevorzugte Alkylierungsmittel sind C₁-C₆-Alkylhalogenide und Di-C₁-C₆-alkylsulfate; außerordentlich bevorzugt Di-C₁-C₆-alkylsulfate.

Ebenso besonders bevorzugte Alkylierungsmittel sind C₁-C₆-Alkylhalogenide, Di-C₁-C₆-alkylsulfate und Di-C₁-C₆-alkylcarbonate; sehr bevorzugt C₁-C₆-Alkylhalogenide und Di-C₁-C₆-alkylsulfate; außerordentlich bevorzugt Di-C₁-C₆-alkylsulfate.

Insbesonders bevorzugte Alkylierungsmittel sind Methylierungsmittel oder Ethylierungsmittel wie Methyliodid, Ethyliodid, Methylbromid, Methylchlorid, Ethylbromid, Ethylchlorid, Dimethylsulfat, Diethylsulfat, C₁-C₆-Alkylsulfonsäuremethylester oder -ethylester oder die Methyl- oder Ethylester der zuvor genannten Phenylsulfonsäuren. Ebenso insbesonders bevorzugte Alkylierungsmittel sind Methylierungsmittel oder Ethylierungsmittel wie Methyliodid, Ethyliodid, Methylbromid, Methylchlorid, Ethylbromid, Ethylchlorid, Dimethylsulfat, Dimethylcarbonat, Diethylsulfat, C₁-C₆-Alkylsulfonsäuremethylester oder -ethylester oder die Methyl- oder Ethylester der zuvor genannten Phenylsulfonsäuren.

Ein ganz besonders bevorzugtes Methylierungsmittel ist Dimethylsulfat.

Im erfindungsgemäßen Verfahren kann man das Alkylierungsmittel sowohl in äquimolarer Menge, bezogen auf die 3-Phenyluracile der Formel II, als auch in substöchiometrischer Menge oder überstöchiometrischer Menge einsetzen.

Üblicherweise setzt man wenigstens eine äquimolare Menge des Alkylierungsmittels III, bezogen auf die 3-Phenyluracile der Formel II ein.

Die molaren Verhältnisse für das Verhältnis von 3-Phenyluracilen der Formel II zu Alkylierungsmittel III liegen in der Regel im Bereich von 1:1 bis 1:3, vorzugsweise 1:1 bis 1:1,3.

Als Basen für die erfindungsgemäße Umsetzung kommen alle üblichen organischen und anorganischen Basen in Betracht.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- oder Erdalkalimetallfluoride wie Cäsiumfluorid, Alkalimetallamide wie Lithiumamid, Natriumamid und Kaliumamid, Alkalimetall- und Erdalkalimetallcarbonate wie Lithiumcarbonat, Natriumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. Ammoniak, primäre Amine wie z.B. Methylamin, Ethylamin, Hexylamin, Anilin, sekundäre Amine wie z.B. Dimethylamin, Diethylamin, tertiäre Amine wie Trimethylamin, Triethylamin, Diisopropylethylamin, Tributylamin, und N-Methylpiperidin, Pyridin, substituierte Pyridine wie Collidin, Lutidin und 4-Dimethylaminopyridin sowie bicyclische Amine wie 1,8-Diazabicyclo[5.4.0]undec-7-en (DBU), 1,5-Diazabicyclo[4.3.0]-non-5-en (DBN) oder 1,4-Diazabicyclo[2.2.2]-octan (DABCO) in Betracht. Bevorzugt sind Basen ausgewählt unter Alkali- und Erdalkalimetallhydroxiden wie Natriumhydroxid, Kaliumhydroxid und Lithiumhydroxid, Alkali- und Erdalkalimetalloxiden wie Calciumoxid, Alkali- und Erdalkalimetallcarbonaten wie Lithiumcarbonat, Natriumcarbonat, Kaliumcarbonat, Cäsiumcarbonat, Magnesiumcarbonat, Calciumcarbonat, Zinkcarbonat, Alkalimetallhydrogencarbonaten wie Natriumhydrogencarbonat sowie Ammoniak oder tertiäre Amine wie Triethylamin;
besonders bevorzugt ausgewählt unter Alkali- und Erdalkalimetallhydroxiden, Ammoniak sowie tertiären Aminen.

Insbesondere bevorzugt sind Basen ausgewählt unter Alkali- und Erdalkalimetallhydroxiden wie Natriumhydroxid, Kaliumhydroxid und Lithiumhydroxid, Alkali- und Erdalkalimetalloxiden wie Calciumoxid, Alkali- und Erdalkalimetallcarbonaten wie Lithiumcarbonat, Natriumcarbonat, Kaliumcarbonat, Cäsiumcarbonat, Magnesiumcarbonat, Calciumcarbonat, Zinkcarbonat sowie Alkalimetallhydrogencarbonaten wie Natriumhydrogencarbonat.

In einer besonders bevorzugten Ausführungsform des erfindungsgemäßen Verfahrens setzt man als Base Natrium- oder Kaliumhydroxid, Natrium- oder Kaliumcarbonat, oder Natriumhydrogen- oder Kaliumhydrogencarbonat ein.

In einer sehr bevorzugten Ausführungsform des erfindungsgemäßen Verfahrens setzt man als Base Alkali- und Erdalkalimetallhydroxide, bevorzugt Alkalimetallhydroxide, ein.

Die Basen werden im allgemeinen in äquimolaren Mengen bezogen auf die 3-Phenyluracile der Formel II eingesetzt, sie können aber auch katalytisch, im Überschuß oder gegebenenfalls als Lösungsmittel verwendet werden.

Vorzugsweise setzt man wenigstens eine äquimolare Menge an Base, bezogen auf die Verbindung II ein. Die Menge an Base wird in der Regel nicht mehr als 1,3 mol bezogen auf 1 mol der Verbindung II betragen.

Bei der erfindungsgemäßen Umsetzung wird der pH-Wert während der gesamten Umsetzung durch portionsweise Basenzugabe in einem Bereich von 1 bis 6 gehalten.

Der pH-Wert wird während der gesamten Umsetzung durch portionsweise Basenzugabe bevorzugt in einem Bereich von 2 bis 6;
sehr bevorzugt von 3 bis 6;
besonders bevorzugt von 4 bis 6;
gehalten.

"Portionsweise Basenzugabe" bedeutet, dass die Basenzugabe während der Umsetzung in einzelnen Portionen erfolgt, d.h. in mindestens 2 Portionen, oder in mehreren bis zu vielen Portionen, oder kontinuierlich.
In besonderen Ausführungsformen des erfindungsgemäßen Verfahrens kann der pH-Wert während der Umsetzung auf verschiedene Art und Weise durch portionsweise Basenzugabe im Bereich von 1 bis 6 gehalten werden, wobei diese Ausführungsformen sowohl für sich alleine betrachtet als auch in Kombination miteinander besondere Ausgestaltungen des erfindungsgemäßen Verfahrens darstellen:

In einer bevorzugten Ausführungsform des erfindungsgemäßen Verfahrens wird zu Beginn der Umsetzung ein pH-Wert zwischen 1 und 6 eingestellt, welcher dann während der Umsetzung konstant auf dem zu Beginn eingestellten Wert gehalten wird.

In einer weiteren bevorzugten Ausführungsform des erfindungsgemäßen Verfahrens wird der zu Beginn der Umsetzung eingestellte pH-Wert zwischen 1 und 6 während der Umsetzung kontinuierlich zu einem anderen pH-Wert im Bereich 1 bis 6 geändert.

In einer weiteren bevorzugten Ausführungsform des erfindungsgemäßen Verfahrens wird die kontinuierliche Änderung des pH-Wertes während der Umsetzung wiederholt,
wobei diese Wiederholung beliebig oft erfolgen kann.

In einer weiteren bevorzugten Ausführungsform des erfindungsgemäßen Verfahrens wird zu Beginn der Umsetzung zunächst ein pH-Wert zwischen 1 und 6 eingestellt, welcher zunächst konstant auf dem eingestellten Wert gehalten wird. Dieser wird dann nach einer Teilumsetzung zu einem anderen pH-Wert im Bereich 1 bis 6 geändert, welcher dann wieder konstant auf dem neu eingestellten Wert gehalten wird. Dieser neu eingestellte pH-Wert kann wiederum nach einer Teilumsetzung zu einem anderen pH-Wert im Bereich 1 bis 6 geändert werden, d. h., die Änderung des eingestellten pH-Werts im Bereich 1 bis 6 nach einer Teilumsetzung kann beliebig oft wiederholt werden. Dies bedeutet, dass der zu Beginn der Umsetzung eingestellte pH-Wert zwischen 1 und 6 ein- oder mehrfach jeweils nach einer Teilumsetzung zu einem anderen pH-Wert im Bereich 1 bis 6 geändert wird, wobei der jeweils geänderte pH-Wert bis zur nächsten Änderung konstant gehalten wird.

In einer insbesonders bevorzugten Ausführungsform des erfindungsgemäßen Verfahrens wird der zu Beginn der Umsetzung eingestellte pH-Wert zwischen 1 und 6 einmal nach einer Teilumsetzung zu einem anderen pH-Wert im Bereich 1 bis 6 geändert.

Als weitere bevorzugte Ausführungsform sind alle Varianten, welche zwischen den voranstehend genannten bevorzugten Ausführungsformen liegen, möglich, wodurch sich auch pH-Wert-Sprünge im Bereich von 1 bis 6 ergeben können.

Alle diese bevorzugten Ausführungsformen können beliebig oft miteinander kombiniert werden und/oder beliebig oft wiederholt werden.

Der zu Beginn der Umsetzung eingestellte pH-Wert zwischen 1 und 6 kann höher liegen als der durch die pH-Wert-Änderung eingestellte pH-Wert bzw. als die durch die pH-Wert-Änderungen eingestellten pH-Werte.

Weiterhin kann der zu Beginn der Umsetzung eingestellte pH-Wert zwischen 1 und 6 niedriger liegen als der durch die pH-Wert-Änderung eingestellte pH-Wert bzw. als die durch die pH-Wert-Änderungen eingestellten pH-Werte.

Darüber hinaus kann der zu Beginn der Umsetzung eingestellte pH-Wert zwischen 1 und 6 zwischen den durch die pH-Wert-Änderungen eingestellten pH-Werten liegen.

Besonders bevorzugt liegt der zu Beginn der Umsetzung eingestellte pH-Wert zwischen 1 und 6 höher als der durch die pH-Wert-Änderung eingestellte pH-Wert bzw. als die durch die pH-Wert-Änderungen eingestellten pH-Werte.

Der pH-Wert kann durch bekannte Routinemethoden, z.B. durch periodische oder kontinuierliche Messung des pH-Wertes, durch den Fachmann ermittelt werden.

Zur Umsetzung können die 3-Phenyluracile der Formel II, die Alkylierungsmittel der Formel III und die Base in an sich beliebiger Weise miteinander in Kontakt gebracht werden, wobei die Zugabe der Base portionsweise erfolgt.

Dies bedeutet, dass die Reaktanden und die Base getrennt, gleichzeitig oder nacheinander in das Reaktionsgefäß eingebracht und zur Reaktion geführt werden können,
wobei die Zugabe der Base portionsweise erfolgt.

Bevorzugt werden die 3-Phenyluracile der Formel II und die Alkylierungsmittel der Formel III in einem Reaktionsgefäß, gegebenenfalls mit dem gewünschten Lösungsmittel, vorlegt und dann durch portionsweise Basenzugabe die gewünschten Reaktionsbedingungen eingestellt.

Man kann jedoch auch die Hauptmenge oder Gesamtmenge an 3-Phenyluracilen der Formel II und die Alkylierungsmittel der Formel III, gegebenenfalls in einem Lösungsmittel, unter Einstellung der gewünschten Reaktionsbedingungen durch portionsweise Basenzugabe in das Reaktionsgefäß eintragen.

Die Umsetzung der 3-Phenyluracile II mit dem Alkylierungsmittel III wird vorteilhaft in Gegenwart eines Lösungsmittels durchgeführt.

Als Lösungsmittel für diese Umsetzungen verwendet man, je nach Temperaturbereich, aliphatische, cycloaliphatische oder aromatische Kohlenwasserstoffe wie Pentan, Hexan, Cyclopentan, Cyclohexan, Toluol, Xylol, chlorierte aliphatische und aromatische Kohlenwasserstoffe wie Dichlormethan, Trichlormethan, 1,2-Dichlorethan, 1,1,2,2-Tetrachlorethan, Chlorbenzol, 1,2-, 1,3- oder 1,4-Dichlorbenzol, Chlortoluole, Dichlortoluole, offenkettige Dialkylether wie Diethylether, Di-n-propylether, Di-isopropylether, Methyl-tert-butylether, cyclische Ether wie Tetrahydrofuran, 1,4-Dioxan, Anisol, Glykolether wie Dimethylglykolether, Diethylglykolether, Diethylenglykoldimethylether, Diethylenglykoldiethylether, C₁-C₄-Alkohole wie Methanol, Ethanol, n-Propanol, iso-Propanol, n-Butanol, aliphatische Carbonsäure-C₁-C₆-alkylester wie Methylacetat, Ethylacetat oder n-Butylacetat; Ketone wie Aceton, Methylethylketon, Methylisopropylketon, Methylisobutylketon, Butanon, Carbonate wie Diethylcarbonat und Ethylencarbonat, N,N-Dialkylamide wie N,N-Dimethylformamid oder N,N-Dimethylacetamid, N-Alkyllactame wie N-Methylpyrrolidon, Sulfoxide wie Dimethylsulfoxid, Tetraalkylharnstoffe wie Tetramethylharnstoff, Tetraethylharnstoff, Tetrabutylharnstoffe, Dimethylethylenharnstoff, Dimethylpropylenharnstoff oder Gemische dieser Lösungsmittel.

Als Lösungsmittel bevorzugt sind N,N-Dimethylformamid, N-Methylpyrrolidon, Aceton, Dichlormethan, Tetrahydrofuran, Toluol, Chlorbenzol, Methylacetat, Ethylacetat, Butylacetat oder Gemische dieser Lösungsmittel.

Vorzugsweise führt man die Alkylierung der 3-Phenyluracile der Formel II bei Temperaturen zwischen -5°C und 100°C, vorzugsweise bei Temperaturen zwischen 0°C und 80°C und insbesondere bei Temperaturen zwischen 20°C und 70°C, sehr bevorzugt zwischen 20°C und 60°C, durch. Die Reaktionszeit kann der Fachmann in an sich üblicher Weise durch Routinemethoden wie Dünnschichtchromatographie oder HPLC ermitteln.

Die Umsetzung kann bei Normaldruck, vermindertem Druck oder unter erhöhtem Druck, gegebenenfalls unter Inertgas kontinuierlich oder diskontinuierlich durchgeführt werden.

Die Aufarbeitung des Reaktionsgemisches zur Gewinnung der 1-Alkyl-3-phenyluracile der Formel I kann nach den hierfür üblichen Methoden erfolgen. In der Regel wird man das verwendete Lösungsmittel nach üblichen Verfahren, beispielsweise destillativ, entfernen. Man kann dann die 1-Alkyl-3-phenyluracile der Formel I in einem mit Wasser nicht mischbaren organischen Lösungsmittel aufnehmen, extrahiert etwaige Verunreinigungen mit gegebenenfalls angesäuertem Wasser, trocknet und entfernt das Lösungsmittel unter vermindertem Druck. Zur weiteren Reinigung kann man die üblichen Verfahren wie Kristallisation, Fällung oder Chromatographie anwenden.

In einer anderen Variante des erfindungsgemäßen Verfahrens kann die Umsetzung auch in einem Mehrphasensystem durchgeführt werden.

Diese Variante des erfindungsgemäßen Verfahrens ist bevorzugt.

Bezüglich Alkylierungsmitteln, pH-Wert, Base, Temperatur, Druck und Aufarbeitung gilt das zuvor gesagte. In der Regel wird man bei Verwendung eines Zweiphasensystems zur Aufarbeitung die Phasen trennen und die Phasen getrennt voneinander nach bekannten Methoden aufarbeiten.

Vorzugsweise wird die Umsetzung in einem wässrig-organischen Mehrphasensystem in Gegenwart von Phasentransferkatalysatoren durchgeführt.
Beispiele für Phasentransferkatalysatoren sind quartäre Ammoniumsalze, Phosphoniumsalze, Kronenether oder Polyglycole.

Geeignete quartäre Ammoniumsalze umfassen z.B.
Tetra-(C₁-C₁₈)-alkylammonium-Fluoride, -Chloride, -Bromide, lodide, -Tetrafluoroborate, -Diborate, Hydrogensulfate, -Hydroxide, -Perchlorate und -Borate wie z.B. Tetramethylammonium-Fluorid-Tetrahydrat, Tetramethylammoniumfluorid, Tetrabutylammoniumfluorid, Tetrabutylammoniumfluorid-Trihydrat, Tetramethylammoniumchlorid, Tetraethylammoniumchlorid, Tetrapropylammoniumchlorid, Tetrabutylammoniumchlorid, Dodecyltrimethylammoniumchlorid, Methyltributylammoniumchlorid, Methyltrioctylammoniumchlorid, Methyltricaprylammonium-Chlorid; Tetramethylammoniumbromid, Tetraethylammonium-Chlorid-Hydrat, Tetraethylammoniumbromid, Tetrapropylammoniumbromid (TPAB), Tetrabutylammoniumbromid (TEAB), Tetrahexylammoniumbromid, Tetraoctylammoniumbromid, Hexadecyltrimethylammoniumbromid (CTAB), Dodecyltrimethylammoniumbromid, Tetrametylammoniumiodid, Tetrabutylammoniumiodid, Tetrahexylammoniumiodid, Tetrabutylammoniumtetrafluoroborat, C₁₂-C₁₄-trimethylammoniumdiborat, Tetrabutylammoniumhydrogensulfat (TBAHS), Tetramethylammoniumhydroxid (TMAOH), Tetraethylammoniumhydroxid, Tetrapropylammoniumhydroxid, Tetrabutylammoniumhydroxid, Tetrabutylammoniumperchlorat, C₁₂-C₁₄-Alkyltrimethylammonium-Borat, C₁₂-C₁₄-Alkyltrimethylammonium-Diborat;
N-Benzyltri-(C₁-C₁₈)-alkylammonium-chloride, -bromide oder -fluoride wie z.B. Benzyltrimethylammoniumchlorid (BTMAC), Benzyltriethylammoniumchlorid (BTEAC), Benzyltriethylammoniumbromid, Benzyltributylammoniumchlorid, Benzyltributylammoniumbromid;
Phenyltri-(C₁-C₁₈)-alkylammonium-chloride, -bromide oder -fluoride wie z.B. Phenyltrimethylammoniumchlorid (PTMAC);
aromatische Ammoniumsalze wie z.B. Hexadecylpyridiniumchlorid, N,N-Dimethylpiperidiniumhydroxid, Pyridinium-Fluoride, -Chloride oder -Bromide wie z.B. 1-Cetyl-Pyridinium-Chlorid-Monohydrat, Cetyl-Pyridinium-Bromid;
vorzugsweise Tetrabutylammoniumchlorid, Methyltributylammoniumchlorid, Methyltrioctylammoniumchlorid; Tetrabutylammoniumbromid, Tetrahexylammoniumbromid, Tetraoctylammoniumbromid, Tetrabutylammoniumiodid, Tetrahexylammoniumiodid, Tetrabutylammoniumhydrogensulfat und Tetrabutylammoniumhydroxid.

Geeignete Phosphoniumsalze umfassen z.B.
C₁-C₁₈-Alkyltriphenylphosphoniumchloride, -bromide, -acetate wie z.B. Methyltriphenylphosphoniumbromid, Ethyltriphenylphosphoniumbromid, Ethyltriphenylphosphoniumiodid, Ethyltriphenylphosphoniumacetat, Butyltriphenylphosphoniumchlorid, Butyltriphenylphosphoniumbromid,
Tetra-(C₁-C₁₈)-alkyl-phosphoniumchlorid oder -bromid wie Tetrabutylphosphoniumbromid,
Tetraphenylphosphoniumchlorid oder -bromid, Benzyltriphenylphosphoniumchlorid oder -bromid.

Geeignete Kronenether umfassen z.B. 18-Krone-6, Dibenzo-18-Krone-6.

Geeignete Polyglycole umfassen z.B. Diethylenglycol-dibutylether (=Butyldiglyme), Tetraethyleneglycol-dimethylether (= Tetraglyme), Triethylenglycol-dimethylether (= Triglyme), Polyglycoldimethylether.

In der Regel setzt man den Phasentransferkatalysator in einer Menge von bis zu 20 mol-%, vorzugsweise zwischen 1 und 15 mol-% und insbesondere zwischen 2 und 12 mol.-%, bezogen auf die 3-Phenyluracile II ein.

Das Mehrphasensystem umfasst eine wässrige Phase und wenigstens eine organische flüssige Phase. Daneben können auch feste Phasen auftreten.
Die wässrige Phase ist bevorzugt eine Lösung von Basen.

Als Basen kommen für diese bevorzugte Variante des erfindungsgemäßen Verfahrens alle üblichen organischen und anorganischen Basen wie voranstehend genannt, insbesondere die voranstehend als bevorzugt bzw. besonders oder sehr bevorzugt genannten Basen in Betracht.

Bevorzugt kommen als Basen Alkalimetall- und Erdalkalimetallhydroxide wie Lithiumhydroxid, Natriumhydroxid, Kaliumhydroxid und Calciumhydroxid; Alkalimetall- und Erdalkalimetallcarbonate wie Lithiumcarbonat, Natriumcarbonat, Kaliumcarbonat und Calciumcarbonat und Alkalimetallhydrogencarbonate wie Natriumhydrogencarbonat, in Betracht.

Besonders bevorzugt verwendet man Alkali- oder Erdalkalimetallhydroxide, sehr bevorzugt Alkalimetallhydroxide wie z.B. Natriumhydroxid.

Die Basen werden im allgemeinen in äquimolaren Mengen bezogen auf die 3-Phenyluracile der Formel II eingesetzt, sie können aber auch katalytisch, im Überschuß oder gegebenenfalls als Lösungsmittel verwendet werden.

Vorzugsweise setzt man wenigstens eine äquimolare Menge an Base, bezogen auf die Verbindung II ein. Die Menge an Base wird in der Regel nicht mehr als 1,3 mol, bezogen auf 1 mol der Verbindung II, betragen.

Die wässrige Phase ist besonders bevorzugt eine Lösung von Basen wie z.B. Alkali- oder Erdalkalimetallhydroxiden, -carbonaten, Alkalimetallhydrogencarbonaten, Ammoniak oder wasserlöslichen primären, sekundären oder tertiären Aminen in Wasser.

Die wässrige Phase ist insbesondere vorzugsweise eine Lösung von Alkali- oder Erdalkalihydroxiden, -carbonaten oder Alkalimetallhydrogencarbonaten in Wasser.

Für die organische Phase kommen vorzugsweise als Lösungsmittel je nach Temperaturbereich aliphatische, cycloaliphatische oder aromatische Kohlenwasserstoffe wie Pentan, Hexan, Cyclopentan, Cyclohexan, Toluol, Xylol, chlorierte aliphatische und aromatische Kohlenwasserstoffe wie Dichlormethan, Trichlormethan, 1,2-Dichlorethan, 1,1,2,2-Tetrachlorethan, Chlorbenzol, 1,2-, 1,3- oder 1,4-Dichlorbenzol, Chlortoluole, Dichlortoluole, offenkettige Dialkylether wie Diethylether, Di-n-propylether, Di-isopropylether, Methyl-tert-butylether, cyclische Ether wie Tetrahydrofuran (THF) und Anisol, aliphatische Carbonsäure-C₁-C₆-alkylester wie Methylacetat, Ethylacetat oder n-Butylacetat oder Gemische dieser Lösungsmittel in Betracht.

Für die organische Phase sind Ethylacetat, n-Butylacetat, Chlorbenzol, THF, Toluol oder Gemische dieser Lösungsmittel als Lösungsmittel bevorzugt; sehr bevorzugt sind Ethylacetat, n-Butylacetat, Chlorbenzol und THF-Gemische dieser Lösungsmittel, sowie Toluol und THF-Gemische von Toluol.

Während der Umsetzung können feste Phasen auftreten, wenn z.B. das 1-Alkyl-3-phenyluracil der Formel I, das 3-Phenyluracil der Formel II, das Alkylierungsmittel der Formel III, die Base und/oder der Phasentransferkatalysator nicht vollständig gelöst sind.

In einer bevorzugten Ausführungsform des erfindungsgemäßen Verfahrens besteht das Mehrphasensystem als wässrige Phase aus wässriger Alkalimetallhydroxid-Lösung, z.B. Natriumhydroxid-Lösung, und als organische Phase aus Toluol und Tetrahydrofuran, oder Dichlormethan und Tetrahydrofuran, Chlorbenzol und Tetrahydrofuran, oder aus Ethylacetat oder n-Butylacetat.

Zur Umsetzung können die 3-Phenyluracile der Formel II, die Alkylierungsmittel der Formel III, die Base und der gegebenenfalls Phasentranferkatalysator in an sich beliebiger Weise miteinander in Kontakt gebracht werden, wobei die Zugabe der Base portionsweise erfolgt.
Die Reaktanden, die Base und gegebenenfalls der Phasentransferkatalysator können getrennt, gleichzeitig oder nacheinander in das Reaktionsgefäß eingebracht und zur Reaktion geführt werden, wobei die Zugabe der Base portionsweise erfolgt.

Beispielsweise können die 3-Phenyluracile der Formel II in einem der vorgenannten organischen Lösungsmittel oder Lösungsmittelgemische vorgelegt werden. Danach gibt man die wässrige Lösung der Base portionsweise, das Alkylierungsmittel III und gegebenenfalls den Phasentransferkatalysator unter Durchmischen zu.

Bevorzugt werden die 3-Phenyluracile der Formel II und die Alkylierungsmittel der Formel III sowie der Phasentransferkatalysator in einem Reaktionsgefäß mit dem gewünschten Lösungsmittel, vorlegt und dann durch portionsweise Basenzugabe die gewünschten Reaktionsbedingungen eingestellt.

Man kann jedoch auch die Hauptmenge oder Gesamtmenge an 3-Phenyluracilen der Formel II und die Alkylierungsmittel der Formel III sowie gegebenenfalls den Phasentransferkatalysator, gegebenenfalls in einem Lösungsmittel, unter Einstellung der gewünschten Reaktionsbedingungen durch portionsweise Basenzugabe in das Reaktionsgefäß eintragen.

Die folgenden Beispiele dienen der Erläuterung der Erfindung.

Unter anderem wurde das Verhältnis von gewünschten 3-Phenyluracilen I zu den entsprechenden dialkylierten Nebenprodukten A bestimmt:

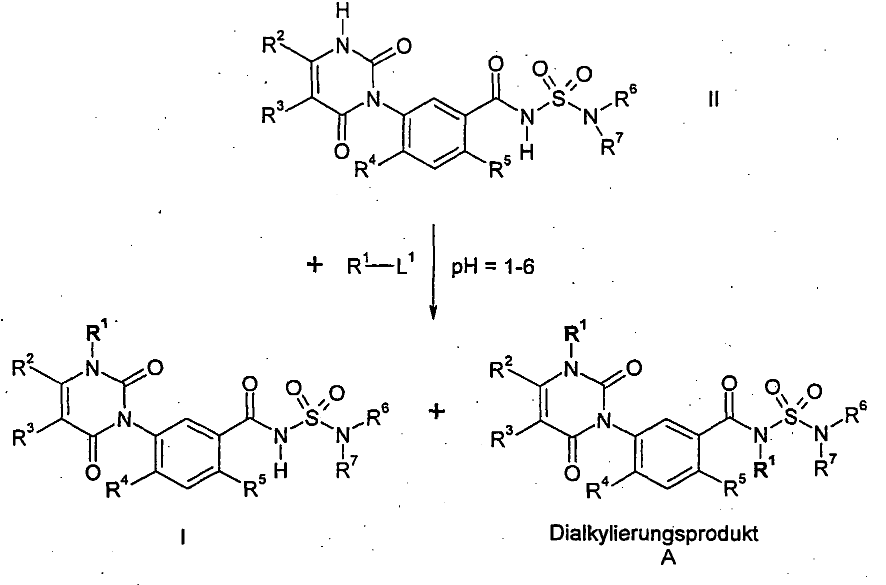

### 1. Erfindungsgemäße Umsetzung bei unterschiedlichen konstanten pH-Werten

12,5 g (24,5 mmol) 2-Chlor-5-[3,6-dihydro-2,6-dioxo-4-(trifluormethyl)-1(2H)-pyrimidinyl]4-fluor-N-{[methyl-(1-methylethyl)amino]sulfonyl}-benzamid, 0,8 g (2,5 mmol) Tetrabutylammoniumbromid (= TBAB) und 3,7 g (29,7 mmol) Dimethylsulfat wurden bei 25 °C in einem Gemisch aus Toluol, Wasser und THF vorgelegt und der Ansatz über 6 h auf 40 °C aufgeheizt. Anschließend stellte man durch Zugabe von wässriger 10 %-iger NaOH-Lösung im Reaktionsgemisch den gewünschten pH-Wert ein.

Während der ganzen Reaktionsdauer wurde weiterhin wässrige 10 %-ige NaOH-Lösung zugegeben, so dass der pH-Wert über den gesamten Reaktionsverlauf konstant bei dem zuvor eingestellten pH-Wert lag.
Nach beendeter Reaktion wurden die Phasen getrennt, die organische Phase getrocknet und das Lösungsmittel entfernt.
Die Ausbeute sowie das Verhältnis von gewünschtem 3-phenyluracil I zu unerwünschtem Dialkylierungsprodukt A wurde über quantitative HPLC (Symmetry C18 5µm 250 x 4,6 mm von Waters; Wellenlänge: 205 nm; Laufmittel: Gradient aus A (0,1 Vol-% H₃PO₄ in H₂O) und B (0,1 Vol-% H₃PO₄ in CH₃CN); B in 25 min von 35% auf 100% ansteigend, dann in 2 min zurück auf 35%; Fluß: 1 ml/min; ) bzw. qualitative HPLC (Symmetry C18 5µm 250 x 4,6 mm von Waters; Wellenlänge: 220 nm; Laufmittel: 40 Gew.-% Acetonitril / 60 Gew.-% Wasser / 0,1 Gew.-% 85-%ige H₃PO₄; Fluß: 1,5 ml/min) bestimmt.

Die Ausbeute an 1-Alkyl-3-phenyluracil I.a.23 (RT: 12,0 min; RT_{Edukt}: 10,0 min) sowie das Verhältnis von gewünschtem 3-Phenyluracil I zu unerwünschtem Dialkylierungsprodukt A mit R¹ = CH₃, R² = CF₃, R³ = H, R⁴ = F, R⁵ = Cl, R⁶ = CH(CH₃)₂, R⁷ = CH₃; im Folgenden "Dialkylierungsprodukt A.a.23" (RT: 13,4 min) bei unterschiedlichen konstanten pH-Werten können Tabelle 2 entnommen werden:

**Tabelle 2**

| Nr. | pH-Wert | Ausbeute [%] | Verhältnis [%] | |
|---|---|---|---|---|
| | | | 1-Alkyl-3-phenyluracil I.a.23 | Dialkylierungsprodukt A.a.23 |
| 2.1 | 4 | 95 | 99,0 | 0,9 |
| 2.2 | 4,5 | | 99,0 | 1,1 |
| 2.3 | 5 | 94 | 98,3 | 1,6 |
| 2.4 | 5,5 | | 96,8 | 3,1 |
| 2.5 | 6 | 95 | 97,0 | 3,2 |
| | | | | |
| 2.6 | 6,5 | 81 | 90,5 | 9,4 |
| 2.7 | 8 | 31 | 51,9 | 48,1 |

### 2. Erfindungsgemäße Umsetzung bei variablem pH-Wert

40,0 g (0,0785 mol) 2-Chlor-5-[3,6-dihydro-2,6-dioxo-4-(trifluormethyl)-1(2H)-pyrimidinyl]4-fluor-N-{[methyl-(1-methylethyl)amino]sulfonyl}-benzamid, 2,5 g (0,0078 mol) Tetrabutylammoniumbromid (= TBAB) und 12,1 g (0,0957 mol) Dimethylsulfat wurden bei 25 °C in einem Gemisch aus Toluol, Wasser und THF vorgelegt und der Ansatz auf 40 °C aufgeheizt. Anschließend stellte man durch Zugabe von wässriger 10 %-iger NaOH-Lösung im Reaktionsgemisch einen pH-Wert von 5,3 - 5,5 ein. Der Ansatz wurde 1 h bei 40 °C gerührt, dabei wurde weiterhin wässrige 10 %-ige Na-OH-Lösung zugegeben, so dass der pH-Wert konstant bei dem zuvor eingestellten pH-Wert lag. Nach 1 h wurde die Zugabe der wässrigen 10 %-ige NaOH-Lösung gestoppt, woraufhin der pH-Wert auf 4,4 - 4,5 fiel. Man gab weitere 0,9 g (0,0071 mol) Dimethylsulfat zu und ließ 10 h bei einem pH-Wert von 4,4 - 4,5 und 40 °C nachrühren. Nach beendeter Reaktion wurden die Phasen getrennt, die organische Phase getrocknet und das Lösungsmittel entfernt.

Die Ausbeute sowie das Verhältnis von gewünschtem 1-Alkyl-3-phenyluracil I.a.23 zu unerwünschtem Dialkylierungsprodukt A.a.23 bei variablem pH-Wert wurden wie unter Versuch 1 genannt bestimmt, und können Tabelle 3 entnommen werden:

**Tabelle 3**

| Nr. | Reakt.zeit [h] | Ausbeute [%] | Verhältnis [%] | |
|---|---|---|---|---|
| | | | 1-Alkyl-3-phenyluracil I.a.23 | Dialkylierungsprodukt A.a.23 |
| 3.1 | 1 | | 99,5 | 0,5 |
| 3.2 | 10 | 92,3 | 98,1 | 1,9 |

### 3. Vergleichsversuch: Einmalige Basenzugabe zu Beginn der Umsetzung

12,5 g (24,5 mmol) 2-Chlor-5-[3,6-dihydro-2,6-dioxo-4-(trifluormethyl)-1 (2H)-pyrimidinyl]4-fluor-N-{[methyl-(1-methylethyl)amino]sulfonyl}-benzamid, 0,8 g (2,5 mmol) Tetrabutylammoniumbromid (= TBAB) und 3,7 g (29,7 mmol) Dimethylsulfat sowie 11,6 g (2,9 mmol) NaOH wurden bei 25 °C in einem Gemisch aus Toluol, Wasser und THF vorgelegt und der Ansatz über 6 h auf 40 °C aufgeheizt. Der pH-Wert betrug am Anfang der Umsetzung 6,3 und am Ende 4,2.
Nach beendeter Reaktion wurden die Phasen getrennt, die organische Phase getrocknet und das Lösungsmittel entfernt.

Die Ausbeute sowie das Verhältnis von gewünschtem 1-Alkyl-3-phenyluracil I.a.23 zu unerwünschtem Dialkylierungsprodukt A.a.23 bei einmaliger Basenzugabe zu Beginn der Umsetzung wurden wie unter Versuch 1 genannt bestimmt, und können Tabelle 4 entnommen werden:

**Tabelle 4**

| Nr. | Ausbeute [%] | Verhältnis [%] | |
|---|---|---|---|
| | | 1-Alkyl-3-phenyluracil I.a.23 | Dialkylierungsprodukt A.a.23 |
| 4.1 | 91 | 94,4 | 5,5 |

### 4. Erfindungsgemäße Umsetzung mit unterschiedlichen Lösungsmitteln

12,5 g (24,5 mmol) 2-Chlor-5-[3,6-dihydro-2,6-dioxo-4-(trifluormethyl)-1(2H)-pyrimidinyl]4-fluor-N-{[methyl-(1-methylethyl)amino]sulfonyl}-benzamid, 0,8 g (2,5 mmol) Tetrabutylammoniumbromid (= TBAB) und 3,7 g (29,7 mmol) Dimethylsulfat wurden bei 25 °C in einem Lösungsmittel oder Lösungsmittelgemisch vorgelegt und der Ansatz auf 40 °C aufgeheizt. Anschließend stellte man durch Zugabe von wässriger 10 %-iger NaOH-Lösung im Reaktionsgemisch einen pH-Wert von 5,3 - 5,5 ein. Während der ganzen Reaktionsdauer wurde weiterhin wässrige 10 %-ige NaOH-Lösung zugegeben, so dass der pH-Wert über den gesamten Reaktionsverlauf konstant bei dem zuvor eingestellten pH-Wert lag.

Nach beendeter Reaktion wurden die Phasen getrennt, die organische Phase getrocknet und das Lösungsmittel entfernt. Das Verhältnis von gewünschtem 1-Alkyl-3-phenyluracil I.a.23 zu unerwünschtem Dialkylierungsprodukt A.a.23 bei unterschiedlichen Lösungsmitteln wurde wie unter Versuch 1 genannt bestimmt, und kann Tabelle 5 entnommen werden:

**Tabelle 5**

| Nr. | Lösungsmittel | Rkt.zeit [h] | Verhältnis [%] | |
|---|---|---|---|---|
| | | | 1-Alkyl-3-phenyluracil I.a.23 | Dialkyl.prod. A.a.23 |
| 5.1 | n-Butylacetat / H₂O | 6 | 97,3 | 2,6 |
| 5.2 | Methylacetat / H₂O | 18 | 98,3 | 1,6 |
| 5.3 | Etylacetat / H₂O | 23 | 97,2 | 2,8 |
| 5.4 | Methylenchlorid / H₂O | 6,5 | 95,5 | 4,5 |
| 5.5 | Ethylformiat / H₂O | 20 | 99,4 | 0,6 |
| 5.6 | Hexylacetat / H₂O | 22 | 98,4 | 1,6 |
| 5.7 | DMSO / H₂O | 23 | 99,5 | <0,5 |
| 5.8 | 2-Methyl-THF / H₂O | 20 | 98,1 | 1,8 |
| 5.9 | DMF / H₂O¹ | 41 | 92,6 | 7,4 |
| 5.10 | Toluol / DMF / H₂O | 20 | 97,3 | 2,6 |
| 5.11 | Toluol / DMF / H₂O | 51 | 96,7 | 3,2 |
| 5.12 | Chlorbenzol / THF / H₂O | 18 | 94,0 | 6,0 |
| 5.13 | Chlorbenzol / THF / H₂O | 20 | 95,0 | 5,0 |
| 5.14 | Chlorbenzol / THF / H₂O | 32 | 97,2 | 2,8 |
| 5.15 | Toluol / H₂O^{2,3} | 22 | 91,9 | 8,1 |
| 5.16 | THF / H₂O² | 48 | 97,9 | 2,1 |

| | | | | |
|---|---|---|---|---|
| ¹ ohne TBAB, Rkt.temp. 40-60°C ² pH = 5,0-5,5 ³ Rkt.temp = 45 °C | | | | |

### 5. Erfindungsgemäße Umsetzung mit unterschiedlichen Alkylierungsmitteln

12,5 g (24,5 mmol) 2-Chlor-5-[3,6-dihydro-2,6-dioxo-4-(trifluormethyl)-1(2H)-pyrimidinyl]4-fluor-N-{[methyl-(1-methylethyl)amino]sulfonyl}-benzamid, 0,8 g (2,5 mmol) Tetrabutylammoniumbromid (= TBAB) und das gewünschte Alkylierungsmittel wurden bei 25 °C in einem Gemisch aus Toluol, Wasser und THF vorgelegt und der Ansatz auf die angegebene Temperatur aufgeheizt. Anschließend stellte man durch Zugabe von wässriger 10 %-iger NaOH-Lösung im Reaktionsgemisch einen pH-Wert von 5,3 - 5,5 ein.

Während der ganzen Reaktionsdauer wurde weiterhin wässrige 10 %-ige NaOH-Lösung zugegeben, so dass der pH-Wert über den gesamten Reaktionsverlauf konstant bei dem zuvor eingestellten pH-Wert lag. Nach beendeter Reaktion wurden die Phasen getrennt, die organische Phase getrocknet und das Lösungsmittel entfernt.

Das Verhältnis von gewünschtem 1-Alkyl-3-phenyluracil I.a.23 zu unerwünschtem Dialkylierungsprodukt A.a.23 bei unterschiedlichen Alkylierungsmitteln wurde wie unter Versuch 1 genannt bestimmt, und kann Tabelle 6 entnommen werden:

**Tabelle 6**

| Nr. | Äqu. Alkylierungsmittel | Rkt.bed. | | Verhältnis [%] | |
|---|---|---|---|---|---|
| | | [h] | [°C] | 1-Alkyl-3-phenylur. I.a.23 | Dialkyl.prod. A.a.23 |
| 6.1 | 1,2 Äqu. Dimethylsulfat | 6 | 40 | 96,8 | 3,1 |
| 6.2 | 1,5 Äqu. Methyliodid¹ | 6 | 40 | 96,2 | 3,8 |
| 6.3 | 1,5 Äqu. Methyliodid | 27 | 40-70 | 94,9 | 5,1 |
| 6.4 | 1,2 Äqu. (H₃CO)₂CO₂ | 36 | 125 | 100 | 0 |

| | | | | | |
|---|---|---|---|---|---|
| ¹ Lösungsmittel = DMF / H₂O | | | | | |

### 6. Erfindungsgemäße Umsetzung mit unterschiedlichen Phasentransferkatalysatoren

125 g (0,24 mol) 2-Chlor-5-[3,6-dihydro-2,6-dioxo-4-(trifluormethyl)-1(2H)-pyrimidinyl]4-fluor-N-{[methyl-(1-methylethyl)amino]sulfonyl}-benzamid (0,02 mol) Phasentransferkatalysator und 37 g (0,30 mol) Dimethylsulfat wurden bei 25 °C in einem Gemisch aus Toluol, Wasser und THF vorgelegt und der Ansatz auf 40 °C aufgeheizt. Anschließend stellte man durch Zugabe von wässriger 10 %-iger NaOH-Lösung im Reaktionsgemisch einen pH-Wert von 5,0 - 5,5 ein. Während der ganzen Reaktionsdauer wurde weiterhin wässrige 10 %-ige NaOH-Lösung zugegeben, so dass der pH-Wert über den gesamten Reaktionsverlauf konstant bei dem zuvor eingestellten pH-Wert lag. Nach beendeter Reaktion wurden die Phasen getrennt, die organische Phase getrocknet und das Lösungsmittel entfernt.

Das Verhältnis von gewünschtem 1-Alkyl-3-phenyluracil I.a.23 zu unerwünschtem Dialkylierungsprodukt A.a.23 bei unterschiedlichen Phasentransferkatalysatoren wurde wie unter Versuch 1 genannt bestimmt, und kann Tabelle 7 entnommen werden:

**Tabelle 7**

| Nr. | Phasentransfer-katalsator | Rkt. zeit [h] | Verhältnis [%] | |
|---|---|---|---|---|
| | | | 1-Alkyl-3-phenylur. I.A.23 | Dial. A.a.23 |
| 7.1 | Tetrabutylammoniumchlorid | 3 | 98,7 | 1,3 |
| 7.2 | Tetrabutylammoniumchlorid | 6 | 97,8 | 2,2 |
| 7.3 | Tetrabutylammoniumbromid | 3 | 98,6 | 1,4 |
| 7.4 | Tetrabutylammoniumbromid | 6 | 97,5 | 2,5 |
| 7.5 | Tetrabutylammoniumhydroxid | 3 | 96,8 | 3,2 |
| 7.6 | Tetrabutylammoniumhydroxid | 6 | 95,1 | 4,9 |
| 7.7 | Tetrabutylammoniumhydrogensulfat | 3 | 97,7 | 2,2 |
| 7.8 | Tetrabutylammoniumhydrogensulfat | 6 | 94,0 | 6,0 |
| 7.9 | Tetrahexylammoniumbromid | 3 | 96,9 | 3,1 |
| 7.10 | Tetrahexylammoniumbromid | 6 | 96,2 | 3,8 |
| 7.11 | Methyltrioctylammoniumchlorid | 3 | 97,3 | 2,7 |
| 7.12 | Methyltrioctylammoniumchlorid | 6 | 95,8 | 4,0 |

### 7. Erfindungsgemäße Umsetzung bei unterschiedlichen Reaktionstemperaturen

12,5 g (24,5 mmol) 2-Chlor-5-[3,6-dihydro-2,6-dioxo-4-(trifluormethyl)-1(2H)-pyrimidinyl]4-fluor-N-{[methyl-(1-methylethyl)amino]sulfonyl}-benzamid, 0,8 g (2,5 mmol) Tetrabutylammoniumbromid (= TBAB) und 3,7 g (29,7 mmol) Dimethylsulfat wurden bei 25 °C in einem Gemisch aus Toluol, Wasser und THF vorgelegt und der Ansatz auf die angegebene Temperatur aufgeheizt. Anschließend stellte man durch Zugabe von wässriger 10 %-iger NaOH-Lösung im Reaktionsgemisch einen pH-Wert von 5,3 - 5,4 ein.
Während der ganzen Reaktionsdauer wurde weiterhin wässrige 10 %-ige NaOH-Lösung zugegeben, so dass der pH-Wert über den gesamten Reaktionsverlauf konstant bei dem zuvor eingestellten pH-Wert lag. Nach beendeter Reaktion wurden die Phasen getrennt, die organische Phase getrocknet und das Lösungsmittel entfernt.

Die Ausbeute sowie das Verhältnis von gewünschtem 1-Alkyl-3-phenyluracil I.a.23 zu unerwünschtem Dialkylierungsprodukt A.a.23 wurde wie unter Versuch 1 genannt bestimmt, und kann Tabelle 8 entnommen werden:

**Tabelle 8**

| Nr. | Temp. [°C] | Ausbeute [%] | Verhältnis [%] | |
|---|---|---|---|---|
| | | | 1-Alkyl-3-Phenyluracil I.a.23 | Dialkylierungsprodukt Aa.23 |
| 8.1 | 30 | 89 | 99,0 | 1,0 |
| 8.2 | 50 | 89 | 98,7 | 1,3 |
| 8.3 | 60 | 86 | 98,4 | 1,6 |
| 8.4 | 70 | 54 | 99,3 | 0,7 |

### 8. Varianz der Zugabe des Methylierungsmittels

### 8a. Zugabe des Methylierungsmittels zu Beginn der Umsetzung

50,0 g (0,98 mol) 2-Chlor-5-[3,6-dihydro-2,6-dioxo-4-(trifluormethyl)-1 (2H)-pyrimidinyl]-4-fluor-N-{[methyl-(1-methylethyl)amino]sulfonyl}-benzamid, 3,2 g (0,0089 mol) Tetrabutylammoniumbromid (= TBAB) und 15,1 g (0,12 mol) Dimethylsulfat wurden bei 25 °C in einem Gemisch aus Toluol, Wasser und THF vorgelegt und der Ansatz auf 40 °C aufgeheizt. Anschließend stellte man durch Zugabe von wässriger 10 %-iger NaOH-Lösung im Reaktionsgemisch einen pH-Wert von 5,3 - 5,5 ein.
Während der ganzen Reaktionsdauer wurde weiterhin wässrige 10 %-ige NaOH-Lösung zugegeben, so dass der pH-Wert über den gesamten Reaktionsverlauf konstant bei dem zuvor eingestellten pH-Wert lag.
Nach beendeter Reaktion wurden die Phasen getrennt, die organische Phase getrocknet und das Lösungsmittel entfernt.

Das Verhältnis von gewünschtem 1-Alkyl-3-phenyluracil I.a.23 zu unerwünschtem Dialkylierungsprodukt A.a.23 wurde wie unter Versuch 1 genannt bestimmt, und kann Tabelle 9 entnommen werden:

**Tabelle 9**

| Nr. | Rkt.zeit [h] | Verhältnis [%] | |
|---|---|---|---|
| | | 1-Alkyl-3-Phenyluracil I.a.23 | Dialkylierungsprodukt A.a.23 |
| 9.1 | 0 | 0 | 0 |
| 9.2 | 0,5 | . 99,4 | 0,6 |
| 9.3 | 1 | 99,4 | 0,6 |
| 9.4 | 1,5 | 99,0 | 0,9 |
| 9.5 | 2 | 98,3 | 1,7 |
| 9.6 | 2,5 | 97,5 | 2,5 |
| 9.7 | 3 | 97,0 | 3,0 |
| 9.8 | 3,5 | 97,0 | 3,0 |

### 8b. Zugabe des Methylierungsmittels portionsweise während der Umsetzung

70,0 g (0,1321 mol) 2-Chlor-5-[3,6-dihydro-2,6-dioxo-4-(trifluormethyl)-1(2H)-pyrimidinyl]-4-fluor-N-{[methyl-(1-methylethyl)amino]sulfonyl}-benzamid, 4,3 g (0,0132 mol) Tetrabutylammoniumbromid (= TBAB) wurden bei 25 °C in einem Gemisch aus Toluol, Wasser und THF vorgelegt und der Ansatz auf 40 °C aufgeheizt. Anschließend stellte man durch Zugabe von wässriger 10 %-iger NaOH-Lösung im Reaktionsgemisch einen pH-Wert von 5,3 - 5,5 ein.
Während der ganzen Reaktionsdauer wurde weiterhin wässrige 10 %-ige NaOH-Lösung zugegeben, so dass der pH-Wert über den gesamten Reaktionsverlauf konstant bei dem zuvor eingestellten pH-Wert lag.
Innerhalb von 8 h wurden 21,0 g (0,17 mol) Dimethylsulfat in Toluol zugetropft.
Nach beendeter Reaktion wurden die Phasen getrennt, die organische Phase getrocknet und das Lösungsmittel entfernt.

Das Verhältnis von gewünschtem 1-Alkyl-3-Phenyluracil I.a.23 zu unerwünschtem Dialkylierungsprodukt A.a.23 wurde wie unter Versuch 1 genannt bestimmt, und kann Tabelle 10 entnommen werden:

**Tabelle 10**

| Nr. | Rkt.zeit [h] | Verhältnis [%] | |
|---|---|---|---|
| | | 1-Alkyl-3-phenyluracil I.a.23 | Dialkylierungsprodukt A.a.23 |
| 10.1 | 8 | 99,0 | 1,0 |
| 10.2 | 12 | 96,9 | 3,0 |

### 9. Erfindungsgemäße Umsetzung mit unterschiedlichen Basen

35,0 g (68,6 mmol) 2-Chlor-5-[3,6-dihydro-2,6-dioxo-4-(trifluormethyl)-1(2H)-pyrimidinyl]4-fluor-N-{[methyl-(1-methylethyl)amino]sulfonyl}-benzamid, 2,2 g (6,9 mmol) Tetrabutylammoniumbromid (= TBAB) und 10,6 g (85,1 mmol) Dimethylsulfat wurden bei 25 °C in einem Lösungsmittel oder Lösungsmittelgemisch vorgelegt und der Ansatz auf 40 °C aufgeheizt. Anschließend stellte man durch Zugabe von 10 %-iger wässriger Lösung der Base im Reaktionsgemisch einen pH-Wert von 5,3 - 5,5 ein. Während der ganzen Reaktionsdauer wurde weiterhin 10 %-ige wässrige Lösung der Base zugegeben, so dass der pH-Wert über den gesamten Reaktionsverlauf konstant bei dem zuvor eingestellten pH-Wert lag.
Nach 4 h wurden die Phasen getrennt, die organische Phase getrocknet und das Lösungsmittel entfernt.

Das Verhältnis von gewünschtem 1-Alkyl-3-Phenyluracil I.a.23 zu unerwünschtem Dialkylierungsprodukt A.a.23 bei Reaktionsführung mit unterschiedlichen Basen wurde wie unter Versuch 1 genannt bestimmt, und kann Tabelle 11 entnommen werden:

**Tabelle 11**

| Nr. | Base | Ausbeute [%] | Verhältnis [%] | |
|---|---|---|---|---|
| | | | 1-Alkyl-3-Phenyluracil I.a.23 | Dialkylierungsprodukt A.a.23 |
| 11.1 | LiOH | 91,8 | 96,6 | 3,4 |
| 11.2 | NaOH | 91,9 | 97,2 | 2,8 |
| 11.3 | KOH | 92,5 | 97,4 | 2,6 |
| 11.4 | NH₃ | 90,5 | 96,8 | 3,2 |
| 11.5 | N(C₂H₅)₃* | | 98,4 | 1,6 |
| 11.6 | DABCO | | 99,6 | 0,4 |

| | | | | |
|---|---|---|---|---|
| * der pH-Wert wurde durch Zugabe von 5%iger wässriger Lösung eingestellt | | | | |

## Patentansprüche

1. Verfahren zur Herstellung von 1-Alkyl-3-phenyluracilen der Formel I 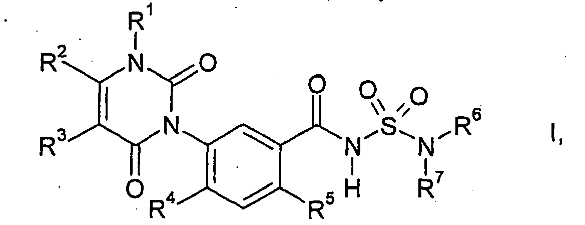 worin die Variablen die folgenden Bedeutungen haben:
R¹ C₁-C₆-Alkyl;
R² und R³ unabhängig voneinander
Wasserstoff, C₁-C₆-Akyl oder C₁-C₆-Halogenalkyl;
R⁴ und R⁵ unabhängig voneinander
Wasserstoff, Halogen, Cyano, C₁-C₆-Alkyl oder C₁-C₆-Halogenalkyl;
R⁶ und R⁷ unabhängig voneinander
Wasserstoff, C₁-C₆-Alkyl, C₁-C₆-Alkoxy, C₃-C₆-Alkenyl, C₃-C₆-Alkynyl, C₃-C₆-Cycloalkyl, C₃-C₆-Cycloalkenyl, Phenyl oder Benzyl;
indem 3-Phenyluracile der Formel II 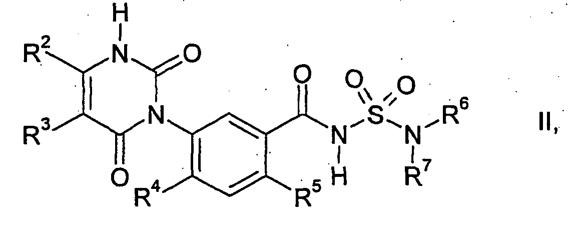 wobei die Variablen R² bis R⁷ die zuvor genannten Bedeutungen haben,
und Alkylierungsmittel der Formel III
R¹-L¹ III,
wobei R¹ die oben genannte Bedeutung hat, und
L¹ für Halogen, Hydrogensulfat, C₁-C₆-Alkylsulfat, C₁-C₆-Alkylcarbonat, C₁-C₆-Alkylsulfonyloxy, C₁-C₆-Halogenalkylsulfonyloxy oder Phenylsulfonyloxy, wobei der Phenylring einen oder mehrere Substituenten aus der Gruppe Halogen, Nitro, C₁-C₆-Alkyl und C₁-C₆-Halogenalkyl tragen kann,
steht;
miteinander umgesetzt werden,
**dadurch gekennzeichnet, dass** während der gesamten Umsetzung der pH-Wert durch portionsweise Basenzugabe in einem Bereich von 1 bis 6 gehalten wird.

2. Verfahren zur Herstellung von 1-Alkyl-3-phenyluracilen der Formel I gemäß Anspruch 1, **dadurch gekennzeichnet, dass** das Alkylierungsmittel ausgewählt ist aus der Gruppe C₁-C₆-Alkylhalogenide, Di-C₁-C₆-alkylsulfate, C₁-C₆-Alkylsulfonsäure-C₁-C₄-alkylester und Phenylsulfonsäure-C₁-C₄-alkylester.

3. Verfahren zur Herstellung von 1-Alkyl-3-phenyluracilen der Formel I gemäß Anspruch 1 oder 2, **dadurch gekennzeichnet, dass** das Alkylierungsmittel ein Di-C₁-C₆-alkylsulfat ist.

4. Verfahren zur Herstellung von 1-Alkyl-3-phenyluracilen der Formel I gemäß einem der Ansprüche 1 bis 3, **dadurch gekennzeichnet, dass** während der gesamten Umsetzung der pH-Wert durch portionsweise Basenzugabe in einem Bereich von 3 bis 6 gehalten wird.

5. Verfahren zur Herstellung von 1-Alkyl-3-phenyluracilen der Formel I gemäß einem der Ansprüche 1 bis 4, **dadurch gekennzeichnet, dass** zu Beginn der Umsetzung ein pH-Wert zwischen 1 und 6 eingestellt wird, welcher dann während der Umsetzung konstant auf dem zu Beginn eingestellten Wert gehalten wird.

6. Verfahren zur Herstellung von 1-Alkyl-3-phenyluracilen der Formel I gemäß einem der Ansprüche 1 bis 5, **dadurch gekennzeichnet, dass** der zu Beginn der Umsetzung eingestellte pH-Wert zwischen 1 und 6 während der Umsetzung kontinuierlich zu einem anderen pH-Wert im Bereich 1 bis 6 geändert wird.

7. Verfahren zur Herstellung von 1-Alkyl-3-phenyluracilen der Formel I gemäß einem der Ansprüche 1 bis 6, **dadurch gekennzeichnet, dass** der zu Beginn der Umsetzung konstant eingestellte pH-Wert zwischen 1 und 6 ein- oder mehrfach jeweils nach einer Teilumsetzung zu einem anderen pH-Wert im Bereich 1 bis 6 geändert wird, wobei der jeweils geänderte pH-Wert bis zur nächsten Änderung konstant gehalten wird.

8. Verfahren zur Herstellung von 1-Alkyl-3-phenyluracilen der Formel I gemäß einem der Ansprüche 1 bis 7, **dadurch gekennzeichnet, dass** die Umsetzung in einem wässrig-organischen Mehrphasensystem in Gegenwart von Phasentransferkatalysatoren durchgeführt wird.

9. Verfahren zur Herstellung von 1-Alkyl-3-phenyluracilen der Formel I gemäß Anspruch 8, **dadurch gekennzeichnet, dass** der Phasentransferkatalysator ausgewählt ist aus der Gruppe quartäre Ammoniumsalze, Phosphoniumsalze, Kronenether und Polyglycole.

10. Verfahren zur Herstellung von 1-Alkyl-3-phenyluracilen der Formel I gemäß Ansprüchen 8 oder 9, **dadurch gekennzeichnet, dass** die wässrige Phase eine Lösung von Alkali- oder Erdalkalihydroxiden, -carbonaten oder Alkalimetallhydrogencarbonaten in Wasser ist.

11. Verfahren zur Herstellung von 1-Alkyl-3-phenyluracilen der Formel I gemäß einem der Ansprüche 1 bis 10, **dadurch gekennzeichnet, dass**
R² C₁-C₄-Halogenalkyl;
R³ Wasserstoff;
R⁴ Wasserstoff oder Fluor;
R⁵ Chlor; und
R⁶ und R⁷ C₁-C₆-Alkyl
bedeuten.

## Claims

1. A process for preparing 1-alkyl-3-phenyluracils of formula I 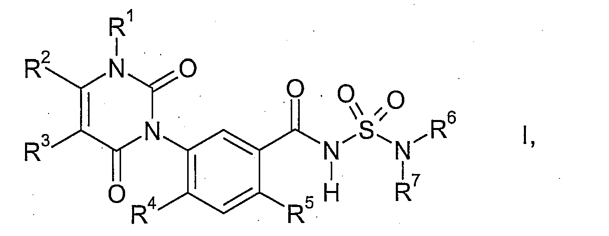 where the variables are as defined below:
R¹ is C₁-C₆-alkyl;
R² and R³ independently of one another are hydrogen, C₁-C₆-alkyl or C₁-C₆-haloalkyl;
R⁴ and R⁵ independently of one another are hydrogen, halogen, cyano, C₁-C₆-alkyl or C₁-C₆-haloalkyl;
R⁶ and R⁷ independently of one another are hydrogen, C₁-C₆-alkyl, C₁-C₆-alkoxy, C₃-C₆-alkenyl, C₃-C₆-alkynyl, C₃-C₆-cycloalkyl, C₃-C₆-cycloalkenyl, phenyl or benzyl;
by reacting 3-phenyluracils of the formula II 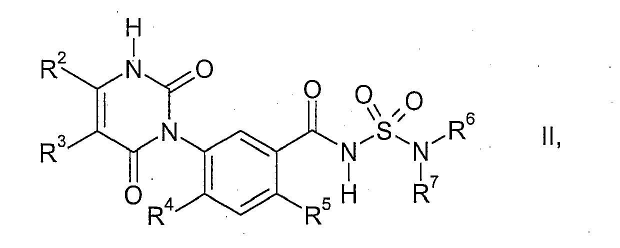 where the variables R² to R⁷ are as defined above
and alkylating agents of the formula III
R¹-L¹ III,
where R¹ is as defined above and
L¹ is halogen, hydrogensulfate, C₁-C₆-alkyl sulfate, C₁-C₆-alkyl carbonate, C₁-C₆-alkylsulfonyloxy, C₁-C₆-haloalkylsulfonyloxy or phenylsulfonyloxy,
where the phenyl ring may carry one or more substituents from the group consisting of halogen, nitro, C₁-C₆-alkyl and C₁-C₆-haloalkyl,
with one another,
wherein during the entire reaction the pH is kept in a range from 1 to 6 by adding base a little at a time.

2. The process for preparing 1-alkyl-3-phenyluracils of the formula I according to claim 1, wherein the alkylating agent is selected from the group consisting of C₁-C₆-alkyl halides, di-C₁-C₆-alkyl sulfates, C₁-C₆-alkyl C₁-C₄-alkylsulfonates and C₁-C₄-alkyl phenylsulfonates.

3. The process for preparing 1-alkyl-3-phenyluracils of the formula I according to claim 1 or 2, wherein the alkylating agent is a di-C₁-C₆-alkyl sulfate.

4. The process for preparing 1-alkyl-3-phenyluracils of the formula I according to any of claims 1 to 3,
wherein during the entire reaction the pH is kept in a range from 3 to 6 by adding base a little at a time.

5. The process for preparing 1-alkyl-3-phenyluracils of formula I according to any of claims 1 to 4,
wherein at the start of the reaction the pH is adjusted to between 1 and 6 and then during the reaction kept constant at the value adjusted at the beginning.

6. The process for preparing 1-alkyl-3-phenyluracils of the formula I according to any of claims 1 to 5,
wherein the pH adjusted during the beginning of the reaction to between 1 and 6 is, during the reaction, changed continuously to another pH in the range of from 1 to 6.

7. The process for preparing 1-alkyl-3-phenyluracils of the formula I according to any of claims 1 to 6,
wherein the pH adjusted constantly during the beginning of the reaction to between 1 and 6 is changed once or more than once, in each case after partial reaction, to another pH in the range of from 1 to 6, the respective changed pH being kept constant until the next change.

8. The process for preparing 1-alkyl-3-phenyluracils of the formula I according to any of claims 1 to 7,
wherein the reaction is carried out in an aqueous/organic multiphase system in the presence of phase-transfer catalysts.

9. The process for preparing 1-alkyl-3-phenyluracils of the formula I according to claim 8, wherein the phase-transfer catalyst is selected from the group consisting of quaternary ammonium salts, phosphonium salts, crown ethers and polyglycols.

10. The process for preparing 1-alkyl-3-phenyluracils of the formula I according to claim 8 or 9, wherein the aqueous phase is a solution of alkali metal or alkaline earth metal hydroxides, carbonates or alkali metal bicarbonates in water.

11. The process for preparing 1-alkyl-3-phenyluracils of the formula I according to any of claims 1 to 10, wherein
R² is C₁-C₄-haloalkyl;
R³ is hydrogen;
R⁴ is hydrogen or fluorine;
R⁵ is chlorine and
R⁶ and R⁷ are C₁-C₆-alkyl.

## Revendications

1. Procédé pour la préparation de 1-alkyl-3-phényluraciles de formule I, 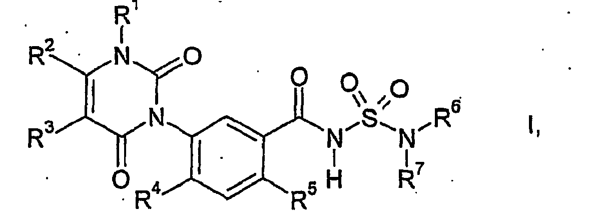 dans laquelle les variables ont les significations suivantes :
R¹ signifie C₁-C₆-alkyle ;
R² et R³ signifient indépendamment l'un de l'autre, hydrogène, C₁-C₆-alkyle ou C₁-C₈-halogénoalkyle
R⁴ et R⁵ représentent, indépendamment l'un de l'autre, hydrogène, halogène, cyano, C₁-C₆-alkyle ou C₁-C₆-halogénoalkyle ;
R⁶ et R⁷ signifie, indépendamment l'un de l'autre, hydrogène, C₁-C₆-alkyle, C₁-C₆-alcoxy, C₃-C₆-alcényle, C₃-C₆-alcynyle, C₃-C₆-cycloalkyle, C₃-C₆-cycloalcényle, phényle ou benzyle ;
en ce qu'on transforme des 3-phényluraciles de formule II 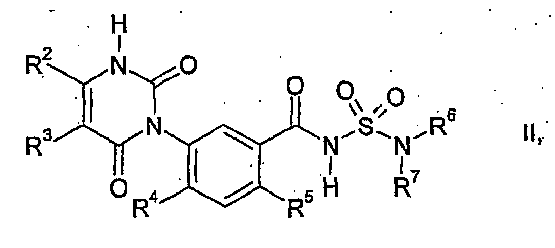 dans laquelle les variables R² à R⁷ ont les significations susmentionnées,
et des agents d'alkylation de formule III
R¹-L¹
où
R¹ a la signification donnée plus haut et
L¹ représente halogène, hydrogénosulfate, C₁-C₆-alkylsulfate, C₁-C₆-alkylcarbonate, C₁-C₆-alkylsulfonyloxy, C₁-C₆-halogénoalkylsulfonyloxy ou phénylsulfonyloxy,
où le cycle phényle peut porter un ou plusieurs substituants du groupe halogène, nitro, C₁-C₆-alkyle et C₁-C₆-halogénoalkyle,
les uns avec les autres,
**caractérisé en ce que,** pendant toute la transformation, le pH est maintenu, par une addition de base, par portions, dans une plage de 1 à 6.

2. Procédé pour la préparation de 1-alkyl-3-phényluraciles de formule I selon la revendication 1,
**caractérisé en ce que** l'agent d'alkylation est choisi dans le groupe formé par les halogénures de C₁-C₆-alkyle, les di-C₁-C₆-alkylsulfates, les esters alkyliques en C₁-C₄ de l'acide C₁-C₆-alkylsulfonique et les esters alkyliques en C₁-C₄ de l'acide phénylsulfonique.

3. Procédé pour la préparation de 1-alkyl-3-phényluraciles de formule I selon la revendication 1 ou 2, **caractérisé en ce que** l'agent d'alkylation est un di-C₁-C₆-alkylsulfate.

4. Procédé pour la préparation de 1-alkyl-3-phényluraciles de formule I selon l'une quelconque des revendications 1 à 3, **caractérisé en ce que,** pendant toute la transformation, le pH est maintenu, par une addition de base, par portions, dans une plage de 3 à 6.

5. Procédé pour la préparation de 1-alkyl-3-phényluraciles de formule I selon l'une quelconque des revendications 1 à 4, **caractérisé en ce qu'**au début de la transformation, on règle un pH entre 1 et 6, qui est ensuite maintenu constant pendant la transformation à la valeur réglée au début.

6. Procédé pour la préparation de 1-alkyl-3-phényluraciles de formule I selon l'une quelconque des revendications 1 à 5, **caractérisé en ce que** le pH réglé entre 1 et 6 au début de la transformation est modifié pendant la transformation en continu à une autre valeur de pH dans la plage de 1 à 6.

7. Procédé pour la préparation de 1-alkyl-3-phényluraciles de formule I selon l'une quelconque des revendications 1 à 6, **caractérisé en ce que** le pH constant réglé au début de la transformation entre 1 et 6 est modifié une ou plusieurs fois, à chaque fois après une transformation partielle, à un autre pH dans la plage de 1 à 6, le pH à chaque fois modifié étant maintenu constant jusqu'à la modification suivante.

8. Procédé pour la préparation de 1-alkyl-3-phényluraciles de formule I selon l'une quelconque des revendications 1 à 7, **caractérisé en ce que** la transformation est réalisée dans un système aqueux-organique à plusieurs phases en présence de catalyseurs de transfert de phase.

9. Procédé pour la préparation de 1-alkyl-3-phényluraciles de formule I selon la revendication 8, **caractérisé en ce que** le catalyseur de transfert de phases est choisi dans le groupe des sels d'ammonium quaternaire, des sels de phosphonium, des éther-couronnes et des polyglycols.

10. Procédé pour la préparation de 1-alkyl-3-phényluraciles de formule I selon les revendications 8
ou 9, **caractérisé en ce que** la phase aqueuse est une solution d'hydroxydes ou de carbonates de métal alcalin
ou alcalino-terreux ou d'hydrogénocarbonates de métal alcalin dans l'eau.

11. Procédé pour la préparation de 1-alkyl-3-phényluraciles de formule I selon l'une quelconque des revendications 1 à 10, **caractérisé en ce que**
R² signifie C₁-C₄-halogénoalkyle ;
R³ signifie hydrogène ;
R⁴ signifie hydrogène ou fluor ;
R⁵ signifie chlore ; et
R⁶ et R⁷ signifient C₁-C₆-alkyle.
